(19) Europäisches Patentamt
European Patent Office
Office européen des brevets

(11) EP 1 581 224 B1

(12) EUROPÄISCHE PATENTSCHRIFT

(45) Veröffentlichungstag und Bekanntmachung des Hinweises auf die Patenterteilung:
**23.01.2008 Patentblatt 2008/04**

(21) Anmeldenummer: **03779979.8**

(22) Anmeldetag: **19.11.2003**

(51) Int Cl.:
***A61K 31/473*** *(2006.01)* ***A61K 31/46*** *(2006.01)*
***A61P 11/06*** *(2006.01)* ***A61P 11/08*** *(2006.01)*

(86) Internationale Anmeldenummer:
**PCT/EP2003/012913**

(87) Internationale Veröffentlichungsnummer:
**WO 2004/050093 (17.06.2004 Gazette 2004/25)**

(54) **TIOTROPIUMHALTIGE ARZNEIMITTELKOMBINATION FUER DIE INHALATION**

TIOTROPIUM-CONTAINING MEDICAMENT COMBINATIONS USED FOR INHALING

PREPARATION MEDICAMENTEUSE A INHALER CONTENANT DU TIOTROPIUM

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **29.11.2002 DE 10256080**

(43) Veröffentlichungstag der Anmeldung:
**05.10.2005 Patentblatt 2005/40**

(73) Patentinhaber: **Boehringer Ingelheim Pharma GmbH & Co.KG**
**55216 Ingelheim am Rhein (DE)**

(72) Erfinder:
- **PAIRET, Michel**
  **88400 BIBERACH (DE)**
- **PIEPER, Michael, P.**
  **88400 BIBERACH (DE)**
- **MEADE, Christopher, John, Montague**
  **88437 Maselheim (DE)**
- **KONETZKI, Ingo**
  **88447 WARTHAUSEN (DE)**

(56) Entgegenhaltungen:
**WO-A-00/75114** **WO-A-01/78739**
**WO-A-01/78741** **WO-A-02/38154**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft neue Arzneimittel zur Inhalation enthaltend ein oder mehrere, bevorzugt ein Tiotropiumsalz in Kombination mit einem oder mehreren pharmakologisch verträglichen Säureadditionssalzen einer Verbindung der Formel **2a**',

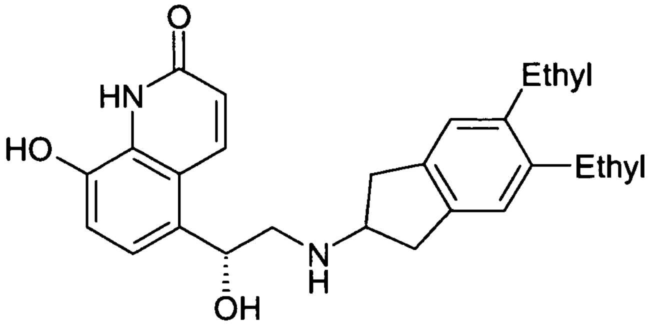

**2a'**,

Verfahren zu deren Herstellung sowie deren Verwendung bei der Therapie von Atemwegserkrankungen.

Hintergrund der Erfindung

Die Verbindung Tiotropiumbromid, ein Salz des Tiotropiums , ist aus der Europäischen Patentanmeldung EP 418 716 A1 bekannt und weist die folgende chemische Struktur auf:

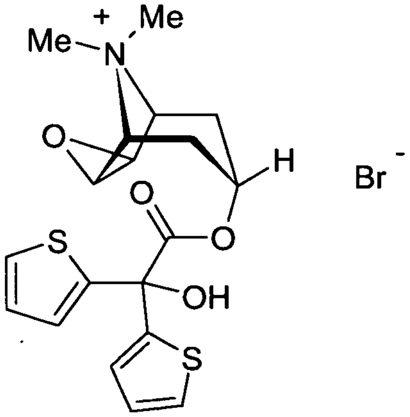

Diese Verbindung kann auch auch durch den chemischen Namen (1α,2β,4β,5α, 7β)-7-[(hydroxydi-2-thienylacetyl)oxy]-9,9-dimethyl-3-oxa-9-azoniatricyclo[3.3.1.0$^{2,4}$] nonanebromid bezeichnet werden und besitzt wertvolle pharmakologische Eigenschaften. Die Bezeichnung Tiotropium ist im Rahmen der vorliegenden Erfindung als Bezugnahme auf das freie Kation **1'** zu verstehen.

Tiotropiumbromid, wie auch andere Salze des Tiotropiums, stellen hochwirksame Anticholinergika dar und können deshalb bei der Therapie von Asthma oder COPD (chronic obstructive pulmonary disease = chronisch obstruktive Lungenerkrankung) einen therapeutischen Nutzen entfalten.

Die Applikation von Tiotropiumsalzen erfolgt vorzugsweise auf inhalativem Wege. Hierbei können geeignete Inhaltionspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten. Die inhalative Applikation kann ferner mittels geeigneter Lösungen des Tiotropiumsalzes erfolgen.

Detaillierte Beschreibung der Erfindung

Überraschenderweise kann ein unerwartet vorteilhafter therapeutischer Effekt, insbesondere ein synergistischer Effekt bei der Behandlung von entzündlichen oder obstruktiven Atemwegserkrankungen beobachtet werden, wenn ein oder mehrere, bevorzugt ein Tiotropiumsalz **1** in Kombination mit pharmakologisch verträglichen Salzen eines Betamimetikums der Formel **2a'**

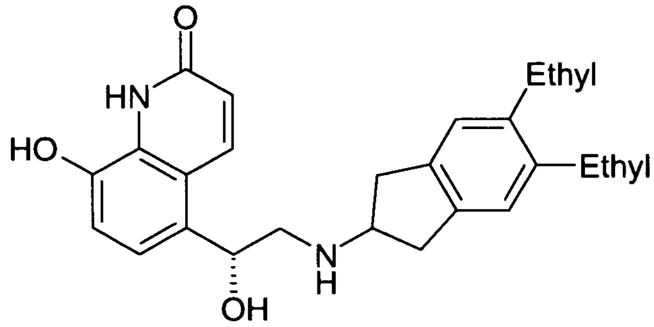

**2a'**

zur Anwendung gelangt.

Hierdurch lassen sich beispielsweise unerwünschte Nebenwirkungen, die häufig bei der Applikation von β-Mimetika am Menschen beobachtet werden, deutlich verringern. Als zentrale Nebenwirkungen von β-Mimetika seien beispielsweise allgemeine Unruhe, Erregung, Schlaflosigkeit, Angst, Fingerzittern, Schweißausbrüche und Kopfschmerzen genannt.

Dementsprechend betrifft die vorliegende Erfindung Arzneimittelkombinationen gekennzeichnet durch den Gehalt an einem oder mehreren, bevorzugt einem Tiotropiumsalz **1** in Kombination mit einem pharmakologisch verträglichen Salz einer Verbindung der Formel **2a'**

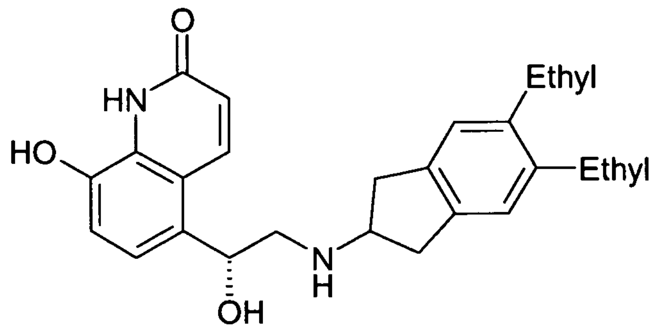

**2a'**.

Die Säureadditionssalze dieser Verbindung werden im folgenden auch als Verbindungen **2a** bezeichnet, während eine Bezugnahme auf die freie Base dieser Verbindung durch die Bezeichnung **2a'** gekennzeichnet ist.

Die Salze **2a** der Verbindung der Formel **2a'** können in die erfindungsgemäßen Arzneimittelkombinationen in Form ihrer Racemate, Enantiomere oder Gemische davon eingesetzt werden. Die Enantiomerentrennung aus den Racematen kann dabei nach im Stand der Technik bekannten Verfahren erfolgen (z.B. durch Chromatographie an chiralen Phasen etc.). Werden die Salze der Verbindung der Formel **2a'** in Form ihrer Enantiomere eingesetzt, gelangen besonders bevorzugt die an der C-OH-Gruppe R-konfigurierten Enantiomere zum Einsatz.

Im Rahmen der vorliegenden Erfindung ist mit der Bezugnahme auf Verbindungen **2a** eine Bezugnahme auf physiologisch verträgliche Säureadditionssalze zu verstehen. Als physiologisch verträgliche Säureadditionssalze **2a** werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Gegebenenfalls können zur Herstellung der Salze **2a** auch Mischungen der vorgenannten Säuren eingesetzt werden.

Erfindungsgemäß bevorzugt sind die Salze **2a** ausgewählt aus der Gruppe bestehend aus Hydrochlorid, Hydrobromid, Sulfat, Phosphat, Fumarat Methansulfonat und Maleat.

Besonders bevorzugt sind die Salze **2a** ausgewählt aus Hydrochlorid und Maleat, von denen das Maleat besonders bevorzugt ist.

Erfolgt im Rahmen der vorliegenden Erfindung eine Bezugnahme auf nicht in der Salzform vorliegende Verbindungen der Formel **2a',** so wird dies durch die Bezeichnung **2a',** wohingegen eine Bezugnahme auf **2a** als Bezugnahme auf die Säureadditionssalze einer Verbindung der Formel **2a'** anzusehen ist. Die Verbindung der Formel **2a'** und **2a** nebst Verfahren zu ihrer Herstellung sind aus der WO 00/75114 bekannt, auf die hiermit inhaltlich Bezug genommen wird.

Die Bezeichnung Tiotropium ist im Rahmen der vorliegenden Erfindung als Bezugnahme auf das freie Kation (**1'**) zu verstehen

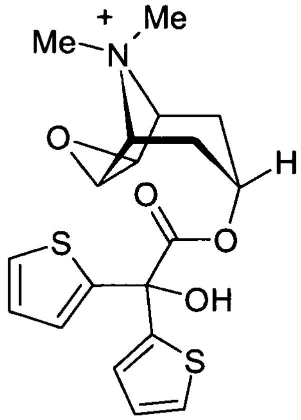

**1'**.

Unter den im Rahmen der vorliegenden Erfindung einsetzbaren Tiotropiumsalzen **1** sind die Verbindungen zu verstehen, die neben Tiotropium als Gegenion (Anion) Chlorid, Bromid, Iodid, Methansulfonat, para-Toluolsulfonat oder Methylsulfat enthalten. Im Rahmen der vorliegenden Erfindung sind von allen Tiotropiumsalzen das Methansulfonat, Chlorid, Bromid oder Iodid bevorzugt, wobei dem Methansulfonat oder dem Bromid besondere Bedeutung zukommt. Von erfindungsgemäß herausragender Bedeutung ist das Tiotropiumbromid. Die Tiotropiumsalze **1** können gegebenenfalls in Form ihrer Solvate und Hydrate zum Einsatz gelangen. Besonders bevorzugt werden dabei die Hydrate verwendet. Von allen Hydraten der erfindungsgemäß einsetzbaren Tiotropiumsalze **1** wird im Rahmen der vorliegenden Erfindung besonders bevorzugt das kristalline Tiotropiumbromidmonohydrat verwendet, welches in der WO 02/30928 beschrieben ist. Auf diese Internationale Patentanmeldung wird an dieser Stelle vollinhaltlich Bezug genommen. Dieses kristalline Tiotropiumbromid-Monohydrat ist gekennzeichnet durch ein bei der thermischen Analyse mittels DSC auftretendes endothermes Maximum bei 230 $\pm$ 5°C bei einer Heizrate von 10K/min. Ferner ist es dadurch gekennzeichnet daß es im IR-Spektrum unter anderem bei den Wellenzahlen 3570, 3410, 3105, 1730, 1260, 1035 und 720 $cm^{-1}$ Banden aufweist. Schließlich weist dieses kristalline Tiotropiumbromidmonohydrat, wie mittels Einkristallröntgenstrukturanalyse bestimmt, eine einfache monoklinische Zelle mit folgenden Dimensionen auf: a = 18.0774 Å, b = 11.9711 Å, c = 9.9321 Å, $\beta$ = 102.691°, V = 2096.96 $Å^3$.

Die erfindungsgemäßen Wirkstoffkombinationen sind überraschenderweise sowohl durch einen raschen Wirkungseintritt, als auch durch eine langandauernde Wirkdauer gekennzeichnet. Dies ist von hoher Bedeutung für das Wohlbefinden des Patienten, da er einerseits nach Applikation der Kombination eine rasche Verbesserung seines Zustands verspürt und andererseits aufgrund der langen Wirkdauer eine einmal pro Tag erfolgende Applikation ausreichend ist.

Die vorstehend genannten Effekte werden sowohl bei gleichzeitiger Applikation innerhalb einer einzigen Wirkstoffformulierung als auch bei sukzessiver Applikation der beiden Wirkstoffe in getrennten Formulierungen beobachtet. Erfindungsgemäß bevorzugt ist die gleichzeitige Applikation der beiden Wirkstoffbestandteile in einer einzigen Formulierung.

Ein Aspekt der vorliegenden Erfindung betrifft ein Arzneimittel, welches ein oder mehrere Tiotropiumsalze **1** sowie Salze **2a** einer Verbindung der Formel **2a'** gegebenenfalls in Form ihrer Solvate oder Hydrate enthält. Hierbei können die Wirkstoffe entweder gemeinsam in einer einzigen Darreichungsform oder in zwei getrennten Darreichungsformen enthalten sein. Erfindungsgemäß bevorzugt sind Arzneimittel, die die Wirkstoffe **1** und **2a** in einer einzigen Darreichungsform enthalten.

Ein weiterer Aspekt der vorliegenden Erfindung betrifft ein Arzneimittel, welches neben therapeutisch wirksamen Mengen von **1** und **2a** einen pharmazeutisch verträglichen Hilfsstoff enthält. Ein Aspekt der vorliegenden Erfindung betrifft ein Arzneimittel, welches neben therapeutisch wirksamen Mengen von **1** und **2a** keinen pharmazeutisch verträglichen Hilfsstoff enthält.

Die vorliegende Erfindung betrifft ferner die Verwendung von **1** und **2a** zur Herstellung eines therapeutisch wirksame Mengen von **1** und **2a** enthaltenden Arzneimittels zur Behandlung von entzündlichen oder obstruktiven Atemwegserkrankungen, insbesondere von Asthma oder COPD durch simultane oder sukzessive Applikation.

Die vorliegende Erfindung zielt ferner auf die simultane oder sukzessive Verwendung therapeutisch wirksamer Dosen der Kombination vorstehender Arzneimittel **1** und **2a** zur Behandlung von entzündlichen oder obstruktiven Atemwegserkrankungen, insbesondere von Asthma oder COPD.

Die Verhältnisse, in denen die beiden Wirkstoffe **1** und **2a** in die erfindungsgemäßen Wirkstoffkombinationen eingesetzt werden können, sind variabel. Die Wirkstoffe **1** und **2a** können gegebenfalls in Form ihrer Solvate oder Hydrate vorliegen. Je nach Wahl der Salze **1** bzw. **2a** variieren die im Rahmen der vorliegenden Erfindung einsetzbaren Gewichtsverhältnisse aufgrund des unterschiedlichen Molekulargewichts der verschiedenen Salzformen. Den nachfolgend angegebenen Gewichtsverhältnissen wurden daher das Tiotropium **1'** sowie die freien Basen **2a'** zugrunde gelegt.

Die erfindungsgemäßen Wirkstoffkombinationen können **1'** und **2a'** in Gewichtsverhältnissen enthalten, die in einem Bereich von 1:300 bis 30:1, bevorzugt von 1:230 bis 20:1, besonders bevorzugt von 1:150 bis 10:1, ferner bevorzugt von 1:50 bis 5:1, besonders bevorzugt von 1:35 bis 2:1.

Beispielsweise und ohne den Umfang der Erfindung darauf zu beschränken, können bevorzugte erfindungsgemäße Kombinationen aus **1** und **2a** Tiotropium **1'** und die besonders bevorzugte Verbindung **2a'** in den folgenden Gewichtsverhältnissen enthalten: 1:40; 1:39; 1:38; 1:37; 1:36; 1:35; 1:34; 1:33; 1:32; 1:31; 1:30; 1:29; 1:28; 1:27; 1:26; 1:25; 1:24; 1:23; 1:22; 1:21; 1:20; 1:19; 1:18; 1:17; 1:16; 1:15; 1:14; 1:13; 1:12; 1:11; 1:10; 1:9; 1:8; 1:7; 1:6; 1:5; 1:4; 1:3; 1:2; 1:1; 2:1; 3:1; 4:1; 5:1.

Die Anwendung der erfindungsgemäßen Arzneimittel enthaltend die Kombinationen aus **1** und **2a** erfolgt üblicherweise so, daß Tiotropium **1'** und **2a**' gemeinsam in Dosierungen von 0,01 bis 10000μg, bevorzugt von 0,1 bis 2000μg, besonders bevorzugt von 10 bis 1000μg, ferner bevorzugt von 15 bis 500μg pro Einmalgabe appliziert werden. Beispielsweise enthalten erfindungsgemäße Kombinationen aus 1 und **2a** eine solche Menge an Tiotropium **1'** und der besonders bevorzugten Verbindung **2a'.** daß die Gesamtdosierung pro Einmalgabe 15μg, 20μg, 25μg, 30μg, 35 μg, 45μg, 50μg, 55μg, 60μg, 65μg, 70μg, 75μg, 80μg, 85μg, 90μg, 95μg, 100μg, 105μg, 110μg, 115μg, 120μg, 125μg, 130μg, 135μg, 140μg, 145μg, 150μg, 155μg, 160μg, 165μg, 170μg, 175μg, 180μg, 185μg, 190μg, 195μg, 200μg, 205μg, 210μg, 215μg, 220μg, 225μg, 230μg, 23μg, 240μg, 245μg, 250μg, 255μg, 260μg, 265μg, 270μg, 275μg, 280μg, 285μg, 290μg, 295μg, 300μg, 305μg, 310μg, 315μg, 320μg, 325μg, 330μg, 335μg, 340μg, 345μg, 350μg, 355μg, 360μg, 365μg, 370μg, 375μg, 380μg, 385μg, 390μg, 395μg 400μg, 405μg ,410μg, 415μg, 420μg, 425μg, 430μg, 435μg, 440μg, 445μg, 450μg, 455μg, 460μg, 465μg, 470μg, 475μg, 480μg, 485μg, 490μg, 495μg, 500μg, 505μg, oder ähnliches beträgt. Bei diesen Dosierungsbereichen sind die Wirkstoffe **1'** und **2a**' in den vorhergehend beschriebenen Gewichtsverhältnissen enthalten.

Beispielsweise und ohne den Umfang der Erfindung darauf zu beschränken, können die erfindungsgemäßen Kombinationen aus **1** und **2a** pro Einmalgabe beispielsweise nachfolgende Mengen an Tiotropium **1'** und **2a'** enthalten: 5μg **1'** und 12,5μg **2a',** 5μg **1'** und 25μg **2a',** 5μg **1'** und 50μg **2a',** 5μg **1'** und 75μg **2a'**, 5μg **1'** und 100μg **2a'**, 5μg **1'** und 200μg **2a',** 10μg **1'** und 12,5μg **2a'**, 10μg **1'** und 25μg **2a',** 10μg **1'** und 50μg **2a',** 10μg **1'** und 75μg **2a',** 10μg **1'** und 100μg **2a',** 10μg **1'** und 200μg **2a',** 18μg **1'** und 12,5 μg **2a',** 18μg **1'** und 25μg **2a',** 18μg **1'** und 50μg **2a',** 18μg **1'** und 75μg **2a',** 18μg **1'** und 100μg **2a'**, 18μg **1'** und 200μg **2a'**, 20μg **1'** und 12,5μg **2a',** 20μg **1'** und 25μg **2a',** 20μg **1'** und 50μg **2a'**, 20μg **1'** und 75μg **2a'**, 20μg **1'** und 100μg **2a',** 20μg **1'** und 200μg **2a',** 36μg **1'** und 12,5μg **2a'**, 36μg **1'** und 25μg **2a'**, 36μg **1'** und 50μg **2a',** 36μg **1'** und 75μg **2a',** 36μg **1'** und 100μg **2a',** 36μg **1'** und 200μg **2a'**, 40μg **1'** und 12,5μg **2a'**, 40μg **1'** und 25μg **2a',** 40μg **1'** und 50μg **2a',** 40μg **1'** und 75μg **2a',** 40μg **1'** und 100μg **2a'** oder 40μg **1**' und 200μg **2a'.**

Wird als erfindungsgemäß bevorzugte Kombination aus **1** und **2a** diejenige Wirkstoffkombination verwendet, in der **1** Tiotropiumbromid bedeutet und in der **2a** für das besonders bevorzugte Salz **2a,** das Maleatsalz der Verbindung **2a**', steht, entsprechen die vorstehend beispielhaft genannten pro Einmalgabe applizierten Wirkstoffmengen von **1'** und **2a'** den nachfolgenden pro Einmalgabe applizierten Mengen an 1 und **2a**: 6μg **1** und 16,2μg **2a**, 6μg **1** und 32,4μg **2a**, 6μg **1** und 64,8μg **2a**, 6μg **1** und 97,2μg **2a**, 6μg **1** und 129,6μg **2a**, 6μg **1** und 259,2μg **2a**, 12μg **1** und 16,2μg **2a**, 12μg **1** und 32,4μg **2a**, 12μg **1** und 64,8μg **2a**, 12μg **1** und 97,2μg **2a**, 12μg **1** und 129,6μg **2a**, 12μg **1** und 259,2μg 2a, 21,7μg **1** und 16,2μg **2a**, 21,7μg **1** und 32,4μg **2a,** 21,7μg **1** und 64,8μg **2a**, 21,7μg **1** und 97,2μg **2a**, 21,7μg **1** und 129,6μg **2a**, 21,7μg **1** und 259,2μg **2a**, 24,1μg **1** und 12,5μg **2a**, 24,1 μg **1** und 32,4μg **2a**, 24,1μg **1** und 64,8μg **2a**, 24,1 μg **1** und 97,2μg **2a,** 24,1 μg **1** und 129,6μg **2a,** 24,1μg **1** und 259,2μg **2a,** 43,3μg **1** und 16,2μg **2a,** 43,3μg **1** und 32,4μg **2a**, 43,3μg **1** und 64,8μg **2a,** 43,3μg **1** und 97,2μg **2a**, 43,3μg **1** und 129,6μg **2a,** 43,3μg **1** und 259,2μg **2a,** 48,1μg **1** und 16,2μg **2a**, 48,1μg **1** und 32,4μg **2a**, 48,1μg **1** und 64,8μg **2a,** 48,1μg **1** und 97,2μg **2a** oder 48,1μg **1** und 129,6μg **2a,** 48,1μg **1** und 259,2μg **2a**.

Wird als erfindungsgemäß bevorzugte Kombination aus **1** und **2a** diejenige Wirkstoffkombination verwendet, in der **1** für das erfindungsgemäß besonders bevorzugte kristalline Tiotropiumbromidmonohydrat steht und in der **2** für das besonders bevorzugte Salz **2a,** das Maleatsalz der Verbindung **2a**', steht, entsprechen die vorstehend beispielhaft genannten pro Einmalgabe applizierten Wirkstoffmengen von **1'** und **2a'** den nachfolgenden pro Einmalgabe applizierten Mengen an 1 und **2a:** 6,2μg **1** und 16,2 μg **2a,** 6,2μg **1** und 32,4μg **2a**, 6,2μg **1** und 64,8μg **2a**, 6,2μg **1** und 97,2μg **2a,** 6,2μg **1** und 129,6μg **2a**, 6,2 μg **1** und 259,2μg **2a**, 12,5 μg **1** und 16,2μg **2a,** 12,5 μg **1** und 32,4μg **2a,** 12,5μg 1 und 64,8μg 2a, 12,5 μg **1** und 97,2μg **2a,** 12,5 μg **1** und 129,6μg 2a, 12,5 μg **1** und 259,2μg **2a**, 22,5μg **1** und 16,2μg

2a, 22,5µg 1 und 32,4µg 2a, 22,5 µg 1 und 64,8µg 2a, 22,5µg 1 und 97,2µg 2a, 22,5µg 1 und 129,6µg 2a, 22,5µg 1 und 259,2µg 2a, 25 µg 1 und 12,5µg 2a, 25 µg 1 und 32,4µg 2a, 25 µg 1 und 64,8µg 2a, 25 µg 1 und 97,2µg 2a, 25 µg 1 und 129,6µg 2a, 25 µg 1 und 259,2µg 2a, 45 µg 1 und 16,2µg 2a, 45 µg 1 und 32,4µg 2a, 45µg 1 und 64,8µg 2a, 45 µg 1 und 97,2µg 2a, 45 µg 1 und 129,6µg 2a, 45 µg 1 und 259,2µg 2a, 50µg 1 und 16,2µg 2a, 50 µg 1 und 32,4µg 2a, 50 µg 1 und 64,8µg 2a, 50 µg 1 und 97,2µg 2a oder 50µg 1 und 129,6µg 2a, 50µg 1 und 259,2µg 2a.

Die Applikation der erfindungsgemäßen Wirkstoffkombinationen aus 1 und 2a erfolgt bevorzugt auf inhalativem Wege. Hierzu müssen die Bestandteile 1 und 2a in inhalierbaren Darreichungsformen bereitgestellt werden.
Als inhalierbare Darreichungsformen kommen insbesondere Inhalationspulver in Betracht. Erfindungsgemäße Inhalationspulver enthaltend die Wirkstoffkombination aus 1 und 2a können allein aus den genannten Wirkstoffen oder aus einem Gemisch der genannten Wirkstoffe mit physiologisch verträglichen Hilfsstoffen bestehen. Die erfindungsgemäßen Darreichungsformen können die Wirkstoffkombination aus 1 und 2a entweder gemeinsam in einer oder in zwei getrennten Darreichungsformen enthalten. Diese im Rahmen der vorliegenden Erfindung bevorzugt einsetzbaren Darreichungsformen werden im nachfolgenden Teil der Beschreibung detailliert beschrieben.

A) Inhalationspulver enthaltend erfindungsgemäße Wirkstoffkombinationen aus 1 und 2a:

Die erfindungsgemäßen Inhaltionspulver können 1- und 2a entweder allein oder im Gemisch mit geeigneten physiologisch unbedenkliche Hilfsstoffen enthalten.

Sind die Wirkstoffe 1 und 2a im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen:

Monosaccharide (z.B. Glucose oder Arabinose), Disaccharide (z.B. Lactose, Saccharose, Maltose, Trehalose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhaltionspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. In besonders bevorzugten Inhalationspulvern ist der Hilfsstoff durch eine mittlere Teilchengröße von 12 bis 35 µm, besonders bevorzugt von 13 bis 30 µm gekennzeichnet. Ferner sind insbesondere solche Inhalationspulver besonders bevorzugt, in denen der 10%-Feinanteil etwa 1 bis 4 µm, bevorzugt etwa 1,5 bis 3 µm beträgt.

Dabei wird unter der mittleren Teilchengröße im hier verwendeten Sinne der 50 % - Wert aus der Volumenverteilung gemessen mit einem Laserdiffraktometer nach der Trockendispersionsmethode verstanden. Analog ist unter dem 10 % - Feinanteil im hier verwendeten Sinne der 10 % - Wert aus der mit einem Laserdiffraktometer gemessenen Volumenverteilung zu verstehen.

Für die erfindungsgemäßen Pulverformulierungen werden bevorzugt Hilfsstoffe hoher Kristallinität verwendet. Diese Kristallinität kann anhand der beim Lösen des Hilfsstoffs freiwerdenden Enthalpie (Lösungsenthalpie) beurteilt werden. Im Falle des erfindungsgemäß besonders bevorzugt zum Einsatz gelangenden Hilfsstoffs Lactosemonohydrat, wird vorzugsweise Lactose verwendet, die durch eine Lösungsenthalpie von ≥ 45 J/g, bevorzugt von ≥ 50 J/g, besonders bevorzugt von ≥ 52 J/g gekennzeichnet ist.

Schließlich wird zur Herstellung der erfindungsgemäßen Inhaltionspulver mikronisierter Wirkstoff 1 und 2a, vorzugsweise mit einer mittleren Teilchengröße von 0,5 bis 10µm, besonders bevorzugt von 1 bis 5µm, der Hilfsstoffmischung beigemischt.

Wird als Wirkstoff 1 das erfindungsgemäß besonders bevorzugte kristalline Tiotropiumbromid-monohydrat eingesetzt, welches durch die WO 02/30928 offenbart wird, hat sich zur Mikronisierung dieser kristallinen Wirkstoffmodifikation insbesondere die nachstehende Vorgehensweise bewährt. Zur Durchführung des Prozesses können gängige Mühlen zum Einsatz gelangen. Bevorzugt wird die Mikronisierung dabei unter Feuchtigkeitsausschluß durchgeführt, besonders bevorzugt unter Einsatz eines entsprechenden Inertgases, wie beispielsweise Stickstoff. Als besonders bevorzugt hat sich die Verwendung von Luftstrahlmühlen erwiesen, in denen die Zerkleinerung des Mahlguts durch Aufeinanderprallen der Partikel miteinender sowie Aufprall der Partikel auf die Wände des Mahlbehälters erfolgt. Als Mahlgas gelangt erfindungsgemäß bevorzugt Stickstoff zur Anwendung. Das Mahlgut wird mittels des Mahlgases unter spezifischen Drücken (Mahldruck) gefördert. Im Rahmen der vorliegenden Erfindung wird der Mahldruck üblicherweise

auf einen Wert zwischen etwa 2 und etwa 8 bar, bevorzugt zwischen etwa 3 und etwa 7 bar, besonders bevorzugt zwischen etwa 3,5 und etwa 6,5 bar eingestellt. Der Eintrag des Mahlgutes in die Luftstrahlmühle erfolgt mittels des Speisegases unter spezifischen Drücken (Speisedruck). Im Rahmen der vorliegenden Erfindung hat sich eine Speisedruck zwischen etwa 2 und etwa 8 bar, bevorzugt zwischen etwa 3 und etwa 7 bar, besonders bevorzugt zwischen etwa 3,5 und etwa 6 bar bewährt. Als Speisegas gelangt vorzugsweise ebenfalls ein Inertgas, besonders bevorzugt ebenfalls Stickstoff zur Anwendung. Die Zufuhr des Mahlguts (kristallines Tiotropiumbromidmonohydrat) kann dabei in einer Förderrate von etwa 5 - 35 g/min, vorzugsweise mit etwa 10-30 g/min erfolgen.
Beispielsweise und ohne den Gegenstand der Erfindung darauf zu beschränken, hat sich als eine mögliche Ausführungsform einer Luftstrahlmühle das folgende Gerät bewährt: 2-Zoll Microniser mit Mahlring 0,8 mm-Bohrung, Firma Sturtevant Inc., 348 Circuit Street, Hanover, MA 02239, USA. Unter Verwendung dieses Geräts wird der Mahlprozess vorzugsweise mit folgenden Mahlparametern durchgeführt:

Mahldruck: etwa 4,5 - 6,5 bar; Speisedruck: etwa 4,5 - 6,5 bar: Zufuhr des Mahlguts: etwa 17-21 g/min.
Das so erhaltene Mahlgut wird anschließend unter den nachfolgend genannten spezifischen Bedingungen weiterverarbeitet. Hierzu wird das Mikronisat bei einer Temperatur von 15 - 40 °C, vorzugsweise 20 - 35 °C, besonders bevorzugt bei 25 - 30 °C Wasserdampf einer relativen Feuchte von wenigstens 40% ausgesetzt. Vorzugsweise wird die Feuchte auf einen Wert von 50 - 95% r.F., bevorzugt auf 60 - 90 % r.F., besonders bevorzugt auf 70 - 80 % r.F. eingestellt.
Unter relativer Feuchte (r.F.) wird hier der Quotient des Partialdruckes des Wasserdampfs und des Dampfdruckes des Wassers bei der betreffenden Temperatur verstanden. Vorzugsweise wird das aus vorstehend beschriebenem Mahlprozess erhältliche Mikronisat den oben genannten Raumbedingungen wenigstens über einen Zeitraum von 6 Stunden ausgesetzt. Bevorzugt wird das Mirkonisat den genannten Raumbedingungen allerdings für etwa 12 bis etwa 48 Stunden, vorzugsweise etwa 18 bis etwa 36 Stunden, besonders bevorzugt etwa 20 bis etwa 28 Stunden ausgesetzt.

Das gemäß vorstehender Vorgehensweise erhältliche erfindungsgemäße Mikronisat des Tiotropiumbromids weist eine mittlere Teilchengröße von zwischen 1,0 μm und 3,5 μm, bevorzugt zwischen 1,1μm und 3,3 μm, besonders bevorzugt zwischen 1,2 μm und 3,0μm und $Q_{(5.8)}$ von größer 60%, bevorzugt größer 70 %, besonders bevorzugt größer 80% auf. Dabei bezeichnet der Kennwert $Q_{(5.8)}$ die Teilchenmenge der Partikel, die bezogen auf die Volumenverteilung der Partikel unterhalb von 5.8 μm liegt. Die Partikelgrößen wurden im Rahmen der vorliegenden Erfindung mittels Laserbeugung (Fraunhoferbeugung) bestimmt. Detailliertere Angaben dazu sind den experimentellen Beschreibungen der Erfindung zu entnehmen.

Ebenso charakteristisch für das erfindungsgemäße Tiotropium-Mikronisat, das nach obigem Prozeß dargestellt wurde, sind spezifische Oberflächenwerte im Bereich zwischen 2 $m^2$/g und 5 $m^2$/g, im besonderen Maße Werte zwischen 2,5 $m^2$/g und 4,5 $m^2$/g und in besonders herausragendem Maße zwischen 3,0 $m^2$/g und 4,0 $m^2$/g.

Nach Einwaage der Ausgangsmaterialien erfolgt die Herstellung der Inhalationspulver aus dem Hilfsstoff und den Wirkstoffen **1** und **2a** unter Verwendung von im Stand der Technik bekannten Verfahren an. Hierbei sei beispielsweise auf die Offenbarung der WO 02/30390 verwiesen.

Die erfindungsgemäßen Inhaltionspulver können entweder in Form einer einzigen Pulvermischung, die sowohl **1** als auch **2a** enthält oder in Form von separaten Inhalationspulvern, die lediglich **1** und **2a** enthalten bereitgestellt und appliziert werden.

Die erfindungsgemäßen Inhalationspulver können mittels aus dem Stand der Technik bekannten Inhalatoren appliziert werden.
Erfindungsgemäße Inhalationspulver, die neben **1** und **2a** ferner einen physiologisch unbedenklichen Hilfsstoff enthalten, können beispielsweise mittels Inhalatoren appliziert werden, die eine einzelne Dosis aus einem Vorrat mittels einer Meßkammer, wie er in der US 4570630A beschrieben wird, oder über andere apparative Vorrichtungen, wie sie in der DE 36 25 685 A beschrieben werden, dosieren. Vorzugsweise werden die erfindungsgemäßen Inhalationspulver, die neben **1** und **2a** physiologisch unbedenkliche Hilfsstoff enthalten, allerdings in Kapseln abgefüllt (zu sogenannten Inhaletten), die in Inhalatoren wie beispielsweise in der WO 94/28958 beschrieben, zur Anwendung gelangen.

Ein zur Anwendung der erfindungsgemäßen Arzneimittelkombination in Kapseln besonders bevorzugter Inhalator ist Figur 1 zu entnehmen.
Dieser Inhalator (Handihaler) für die Inhalation pulverförmiger Arzneimittel aus Kapseln ist gekennzeichnet durch ein Gehäuse 1, enthaltend zwei Fenster 2, ein Deck 3, in dem sich Lufteinlaßöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 8 vorgesehen ist, sowie ein über eine Achse 10 klappbar mit dem Gehäuse 1, dem Deck 3 und einer Kappe 11 verbundenes Mundstück 12, sowie Luftdurchlaßlöcher 13 zur Einstellung des Strömungswiderstandes.

Für die Anwendung der erfindungsgemäßen Inhalationspulver enthaltend **1** und **2a** mittels pulverhaltiger Kapseln werden besonders bevorzugt solche Kapseln verwendet, deren Material ausgewählt ist aus der Gruppe der synthetischen Kunststoffe, besonders bevorzugt ausgewählt aus der Gruppe bestehend aus Polyethylen, Polycarbonat, Polyester, Polypropylen und Polyethylenterephthalat. Besonders bevorzugt sind als synthetische Kunststoffmaterialien Polyethylen, Polycarbonat oder Polyethylenterephthalat. Wird Polyethylen als eines der erfindungsgemäß besonders bevorzugten Kapselmaterialien verwendet, gelangt vorzugsweise Polyethylen mit einer Dichte zwischen 900 und 1000 $kg/m^3$, bevorzugt von 940 - 980 $kg/m^3$ , besonders bevorzugt von etwa 960 - 970 $kg/m^3$ (high-density Polyethylen) zur Anwendung.
Die synthetischen Kunststoffe im Sinne der Erfindung können vielseitig mittels dem im Stand der Technik bekannten Herstellverfahren verarbeitet werden. Bevorzugt im Sinne der Erfindung wird die spritzgusstechnische Verarbeitung der Kunststoffe. Besonders bevorzugt wird die Spritzgusstechnik unter Verzicht auf die Verwendung von Formtrennmitteln. Dieses Herstellverfahren ist wohldefiniert und durch eine besonders gute Reproduzierbarkeit gekennzeichnet.

Ein weiterer Aspekt der vorliegenden Erfindung betrifft vorstehend genannte Kapseln, die vorstehend genannte erfindungsgemäße Inhalationspulver mit **1** und **2a** enthalten. Sollen die erfindungsgemäßen Inhalationspulver im Sinne der vorstehend gennannten bevorzugten Anwendung in Kapseln (Inhaletten) abgefüllt werden, bieten sich Füllmengen von 1 bis 30mg, bevorzugt von 3 bis 20mg, bevorzugt 5 bis 10 mg Inhalationspulver pro Kapsel an. Diese enthalten erfindungsgemäß entweder gemeinsam oder jeweils die bereits vorstehend für **1** und **2a** genannten Dosierungen pro Einmalgabe. Wie bereits vorstehend erläutert umfaßt die vorliegende Erfindung ferner ein Kit bestehend aus zwei Kapseln, die jeweils einen der Wirkstoffe **1** und **2a** gegebenenfalls in Kombination mit einem der vorstehend genannten physiologisch verträglichen Hilfsstoffe enthalten.

Ferner betrifft die vorliegende Erfindung ein Inhalationskit, bestehend aus einer oder mehrerer der vorstehend beschriebenen, durch einen Gehalt an erfindungsgemäßem Inhalationspulver mit **1** und **2a** gekennzeichneten Kapseln in Verbindung mit dem Inhalator gemäß Figur 1.

Die vorliegende Erfindung betrifft ferner die Verwendung der vorstehend genannten, durch einen Gehalt an erfindungsgemäßem Inhalationspulver mit 1 und 2a gekennzeichneten Kapseln, zur Herstellung eines Arzneimittels zur Behandlung von Atemwegserkrankungen, insbesondere zur Behandlung von COPD und/oder Asthma.

Die Darstellung von befüllten Kapseln, die die erfindungsgemäßen Inhalationspulver enthalten, erfolgt nach im Stand der Technik bekannten Verfahren durch Befüllung der leeren Kapseln mit den erfindungsgemäßen Inhalationspulvern.

B) Treibgashaltige Inhalationsaerosole enthaltend erfindungsgemäße Wirkstoffkombinationen aus **1** und **2a**:

Erfindungsgemäße treibgashaltige Inhaltionsaerosole können **1** und **2a** im Treibgas gelöst oder in dispergierter Form enthalten. Hierbei können **1** und **2a** in getrennten Darreichungsformen oder in einer gemeinsamen Darreichungsform enthalten sein, wobei 1 und **2a** entweder beide gelöst, beide dispergiert oder jeweils nur eine Komponente gelöst und die andere dispergiert enthalten sein können.
Die zur Herstellung der erfindungsgemäßen Inhaltionsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus TG 134a und TG227 und Mischungen derselben.

Die erfindungsgemäßem treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die erfindungsgemäßen treibgashaltigen Inhaltionsaerosole können bis zu 5 Gew-% an Wirkstoff **1** und/oder **2a** enthalten. Erfindungsgemäße Aerosole enthalten beispielsweise 0,002 bis 5 Gew-%, 0,01 bis 3 Gew-%, 0,015 bis 2 Gew-%, 0,1 bis 2 Gew-%, 0,5 bis 2 Gew-% oder 0,5 bis 1 Gew-% an Wirkstoff **1** und/oder **2a**.

Liegen die Wirkstoffe **1** und/oder **2a** in dispergierter Form vor weisen die Wirkstoffteilchen bevorzugt eine mittlere Teilchengröße von bis zu 10 μm, bevorzugt von 0,1 bis 5 μm, besonders bevorzugt von 1 bis 5 μm auf. Soll **1** in Form seines kristallinen Tiotropiumbromid-monohydrats zum Einsatz gelangen, kann dieses gegebenenfalls in Form des im vorstehenden Abschnitt detaillierter beschriebenen Mikronisats zum Einsatz gelangen.

Die vorstehend genannten erfindungsgemäßen treibgashaltigen Inhaltionaerosole können mittels im Stand der Technik bekannten Inhalatoren (MDIs = metered dose inhalers) appliziert werden. Dementsprechend betrifft ein weiterer Aspekt der vorliegenden Erfindung Arzneimittel in Form von wie vorstehend beschriebenen treibgashaltigen Aerosolen in Verbindung mit einem oder mehreren zur Verabreichung dieser Aerosole geeigneten Inhalatoren. Ferner betrifft die vorliegende Erfindung Inhalatoren, dadurch gekennzeichnet, daß sie vorstehend beschriebene erfindungsgemäße treibgashaltige Aerosole enthalten.
Die vorliegende Erfindung betrifft ferner Kartuschen, die ausgestattet mit einem geeigneten Ventil in einem geeigneten

Inhalator zur Anwendung gelangen können und die eine der vorstehend genannten erfindungsgemäßen treibgashaltigen Inhalationsaerosole enthalten. Geeignete Kartuschen und Verfahren zur Abfüllung dieser Kartuschen mit den erfindungsgemäßen treibgashaltigen Inhaltionsaerosolen sind aus dem Stand der Technik bekannt.

C) Treibgasfreie Inhaltionslösungen oder Suspensionen enthaltend erfindungsgemäße Wirkstoffkombinationen aus **1** und **2a:**

Besonders bevorzugt erfolgt die Applikation der erfindungsgemäßen Wirkstoffkombination in Form von treibgasfreien Inhalationslösungen und Inhaltionssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die **1** und **2a ,** getrennt oder gemeinsam enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Erfindungsgemäß kann in der vorliegenden Formulierung auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/ 100ml, besonders beovorzugt unter 20 mg/ 100ml. Generell sind solche Inhaltionslösungen bevorzugt, in denen der Gehalt an Natriumedetat bei 0 bis 10mg/100ml liegt.

Den erfindungsgemäßen treibgasfreien Inhaltionslösungen können Co-Solventien und/oder weitere Hilfsstoffe zugesetzt werden.

Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.

Unter Hilfs- und Zusatzstoffen wird in diesem Zusammenhang jeder pharmako logisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu denZusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/ 100ml, besonders bevorzugt zwischen 5 und 20 mg/100ml enthalten.

Bevorzugte Formulierungen enthalten außer dem Lösungsmittel Wasser und der Wirkstoffkombination aus **1** und **2a** nur noch Benzalkoniumchlorid und Natriumedetat.

In einer anderen bevorzugten Ausführungsform wird auf Natriumedetat verzichtet.

Zur Applikation der erfindungsgemäßen treibgasfreien Inhaltionslösungen sind besonders solche Inhalatoren, die eine kleine Menge einer flüssigen Formulierung in der therapeutisch notwendigen Dosierung binnen weniger Sekunden in ein therapeutischinhalativ geeignetes Aerosol vernebeln können. Im Rahmen der vorliegenden Erfindung sind solche Vernebler bevorzugt, bei denen bereits eine Menge von weniger als 100 μL, bevorzugt weniger als 50 μL, besonders bevorzugt zwischen 20 und 30 μL Wirkstofflösung mit bevorzugt einem Hub zu einem Aerosol mit einer durchschnittlichen Teilchengröße von weniger als 20 μm, bevorzugt weniger als 10 μm, so vernebelt werden können, daß der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 als auch in der WO 97/12687 (dort insbesondere Figuren 6a und 6b) ausführlich beschrieben. Die dort beschriebenen Vernebler (Devices) sind auch unter der Bezeichnung Respimat® bekannt.

Dieser Vernebler (Respimat®) kann vorteilhaft zur Erzeugung der erfindungsgemäßen inhalierbaren Aerosole enthaltend die Wirkstoffkombination aus 1 und 2a eingesetzt werden. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so daß inhalierbare Aerosole entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch

- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

Der Hohlkolben mit Ventilkörper entspricht einer in der WO 97/12687 offenbarten Vorrichtung. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 Mpa (etwa 50 bis 600 bar), bevorzugt 10 bis 60 Mpa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-94/07607 offenbart; auf diese Schrift wird hiermit inhaltlich Bezug genommen, insbesondere auf die dort offenbarte Figur 1 und deren Beschreibung.

Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt.

Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°.

Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.

Die Strahlrichtungen treffen sich dementsprechend in der Umgebung der Düsenöffnungen.

Die flüssige Arzneimittelzubereitung trifft mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchen- bzw. Tröpfchengrößen des Aerosols liegen bei bis zu 20 Mikrometern, bevorzugt 3 bis 10 Mikrometern.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseobereil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

In den dieser Patentanmeldung beigefügten Figuren 2a/b, die identisch sind mit den Figuren 6 a/b der WO 97/12687, ist der Vernebler (Respimat®) beschrieben, mit dem die erfindungsgemäßen wäßrigen Aerosolzubereitungen vorteilhaft inhaliert werden können.

Figur 2a zeigt einen Längsschnitt durch den Zerstäuber bei gespannter Feder, Figur 2b zeigt einen Längsschnitt durch den Zerstäuber bei entspannter Feder.

Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseobereil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

Wird die erfindungsgemäße Formulierung mittels der vorstehend beschriebenen Technik (Respimat®) vernebelt, sollte die ausgebrachte Masse bei wenigstens 97%, bevorzugt wenigstens 98% aller Betätigungen des Inhalators (Hube) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann jedoch auch mittels anderer als der vorstehend beschriebenen Inahalatoren, beispielsweise Jet-Stream-Inhalatoren, vernebelt werden.

Dementsprechend betrifft ein weiterer Aspekt der vorliegenden Erfindung Arzneimittel in Form von wie vorstehend beschriebenen treibgasfreien Inhaltionslösungen oder Suspensionen in Verbindung mit einer zur Verabreichung dieser Formulierungen geeigneten Vorrichtung, bevorzugt in Verbindung mit dem Respimat®. Bevorzugt zielt die vorliegende Erfindung auf treibgasfreie Inhaltionslösungen oder Suspensionen gekennzeichnet durch die erfindungsgemäßen Wirkstoffkombination aus 1 und 2a in Verbindung mit der unter der Bezeichnung Respimat® bekannten Vorrichtung. Ferner betrifft die vorliegende Erfindung vorstehend genannte Vorrichtungen zur Inhalation, bevorzugt den Respimat®, dadurch gekennzeichnet, daß sie vorstehend beschriebene erfindungsgemäße treibgasfreie Inhaltionslösungen oder Suspensionen enthalten.

Die erfindungsgemäßen treibgasfreien Inhalationslösungen oder Suspensionen können neben den vorstehend, zur Applikation im Respimat vorgesehenen Lösungen und Suspensionen auch als Konzentrate oder sterile gebrauchsfertige Inhalationslösungen bzw. -suspensionen vorliegen. Aus den Konzentraten lassen sich beispielsweise durch Zugabe von isotonischen Kochsalzlösungen gebrauchsfertige Formulierungen generieren. Sterile gebrauchsfertige Formulierungen können mittels energiebetriebener Stand- oder transportabler Vernebler, die inhalierbare Aerosole mittels Ultraschall oder Druckluft nach dem Venturiprinzip oder anderen Prinzipien erzeugen, appliziert werden.

Dementsprechend betrifft ein weiterer Aspekt der vorliegenden Erfindung Arzneimittel in Form von wie vorstehend beschriebenen treibgasfreien Inhaltionslösungen oder Suspensionen, die als Konzentrate oder sterile gebrauchsfertige Formulierungen vorliegen, in Verbindung mit einer zur Verabreichung dieser Lösungen geeigneten Vorrichtung, dadurch gekennzeichnet, daß es sich bei dieser Vorrichtung um einen energiebetriebenen Stand- oder transportablen Vernebler handelt, der inhalierbare Aerosole mittels Ultraschall oder Druckluft nach dem Venturiprinzip oder anderen Prinzipien erzeugt.

Die folgenden Beispiele dienen einer weitergehenden Erläuterung der vorliegenden Erfindung

**Ausgangsmaterialien:**

**I) Mikronisierung von kristallinem Tiotropiumbromid-monohydrat:**

Das gemäß der WO 02/30928 erhältliche kristalline Tiotropiumbromid-monohydrat wird mit einer Luftstrahlmühle vom Typ 2-Zoll Microniser mit Mahlring 0,8 mm-Bohrung, Firma Sturtevant Inc., 348 Circuit Street, Hanover, MA 02239, USA mikronisiert. Unter Verwendung von Stickstoff als Mahlgas werden dabei beispielsweise die folgenden Mahlparameter eingestellt:

Mahldruck: 5,5 bar; Speisedruck: 5,5 bar;
Zufuhr (des kristallinen Monohydrats) bzw. Fließgeschwindigkeit: 19 g/min.

Das erhaltene Mahlgut wird anschließend auf Hordenblechen in einer Schichtdicke von etwa 1 cm ausgebreitet und für 24 - 24,5 Stunden den folgenden Klimabedingungen unterworfen:

Temperatur. 25 - 30 °C; Relative Feuchte: 70-80%.

**Meßmethoden:**

**I) Partikelgrößenbestimmung von Tiotropium Monohydrat, mikronisiert:**

Messgerät und Einstellungen:

Die Bedienung der Geräte erfolgte in Übereinstimmung mit den Bedienungsanleitungen des Herstellers.

| | |
|---|---|
| Messgerät: | Laser-Beugungs-Spektrometer (HELOS), Sympatec |
| Dispergiereinheit: | Trockendispergierer RODOS mit Saugtrichter,Sympatec |
| Probenmenge: | 200 mg $\pm$ 150 mg |
| Produktzufuhr: | Schwingrinne Vibri, Fa. Sympatec |
| Frequenz d. Vibrationsrinne: | bis 100 % ansteigend |

(fortgesetzt)

| | |
|---|---|
| Dauer der Probenzufuhr: | 15 bis 25 sek. (im Fall von 200 mg) |
| Brennweite: | 100 mm (Messbereich: 0,9 - 175 μm) |
| Messzeit/Wartezeit: | ca. 15 s (im Fall von 200 mg) |
| Zykluszeit: | 20 ms |
| Start/Stop bei: | 1 % auf Kanal 28 |
| Dispergiergas: | Druckluft |
| Druck: | 3 bar |
| Unterdruck: | maximal |
| Auswertemodus: | HRLD |

Probenvorbereitung / Produktzufuhr:

Ca. 200 mg der Prüfsubstanz werden auf einem Kartenblatt eingewogen.
Mit einem weiteren Kartenblatt werden alle größeren Agglomerate zerstoßen.
Das Pulver wird dann auf der vorderen Hälfte der Schwingrinne (ab ca. 1 cm vom vorderen Rand) fein verteilt aufgestreut.
Nach dem Start der Messung wird die Frequenz der Schwingrinne so variiert, dass die Zufuhr der Probe möglichst kontinuierlich erfolgt. Die Produktmenge darf aber auch nicht zu groß sein damit eine ausreichende Dispergierung erreicht wird.

**II) Partikelgrößenbestimmung des Hilfsstoffs (hier Laktose):**

Messgerät und Einstellungen

Die Bedienung der Geräte erfolgte in Übereinstimmung mit den Bedienungsanleitungen des Herstellers.

| | |
|---|---|
| Messgerät: | Laser-Beugungs-Spektrometer (HELOS), Sympatec |
| Dispergiereinheit: | Trockendispergierer RODOS mit Saugtrichter, Sympatec |
| Probenmenge: | 200 mg ± 100 mg |
| Produktzufuhr: | Vibrationsrinne Typ VIBRI , Sympatec |
| Frequenz d. Vibrationsrinne: | 100 % ansteigend |
| Brennweite: | 200 mm (Messbereich: 1,8 - 350 μm) |
| Messzeit/Wartezeit: | ca. 10 s (im Falle von 200 mg) |
| Zykluszeit: | 10 ms |
| Start/Stop bei: | 1 % auf Kanal 28 |
| Dispergiergas: | Druckluft |
| Druck: | 3 bar |
| Unterdruck: | maximal |
| Auswertemodus: | HRLD |

Probenvorbereitung / Produktzufuhr:

Ca. 200 mg der Prüfsubstanz werden auf einem Kartenblatt eingewogen. Mit einem weiteren Kartenblatt werden alle größeren Agglomerate zerstoßen. Das Pulver wird in die Vibrationsrinne überführt. Es wird ein Abstand von 1.2 bis 1.4 mm zwischen Vibrationsrinne und Trichter eingestellt. Nach dem Start der Messung wird die Amplitudeneinstellung der Schwingrinne möglichst kontinuierlich auf 100 % gegen Ende der Messung gesteigert.

**III) Bestimmung der spezifischen Oberfläche von Tiotropiumbromid-Monohydrat, mikronisiert (1-Punkt-BET-Methode):**

Prinzip

Die Bestimmung der spezifischen Oberfläche erfolgt, indem die Pulverprobe einer Stickstoff/Heliumatmosphäre bei unterschiedlichen Drücken ausgesetzt wird. Durch Abkühlung der Probe erfolgt eine Kondensation der Stickstoffmolekülen auf der Oberfläche der Partikel. Die kondensierte Stickstoffmenge wird über die Änderung der thermischen Leitfähigkeit des Stickstoff/Heliumgemisches bestimmt und die Oberfläche der Probe über den Flächenbedarf von Stickstoff bestimmt. Über diesen Wert und die Probeneinwaage wird die spezifische Oberfläche berechnet.

Messgerät und Einstellungen

| | |
|---|---|
| Messgerät: | Monosorb, Fa. Quantachrome |
| Ausheizgerät: | Monotektor, Fa. Quantachrome |
| Mess- und Trockengas: | Stickstoff (5.0) / Helium (4.6) 70/30, Fa. Messer Griesheim |
| Adsorbat: | Stickstoff 30%-ig in Helium |
| Kältemittel: | flüssiger Stickstoff |
| Messzelle: | mit Kapillarrohr, Fa. W. Pabisch GmbH&Co.KG |
| Kalibrierspritze; | 1000 µl, Fa. Precision Sampling Corp. |
| Analysenwaage: | R 160 P, Fa. Satorius |

Berechnung der spezifischen Oberfläche:

Die Messwerte werden vom Gerät in [m$^2$] angezeigt und werden i.d.R. in [cm$^2$/g] auf die Einwaage (Trockenmasse) umgerechnet:

$$A_{spez} = \frac{MW * 10000}{m_{tr}}$$

| | |
|---|---|
| $A_{spez}$ = | spezifische Oberfläche [cm$^2$/g] |
| MW = | Messwert [m$^2$] |
| $m_{tr}$ = | Trockenmasse [g] |
| 10000 = | Umrechnungsfaktor [cm$^2$/m$^2$] |

**IV) Bestimmung der Lösungswärme der Laktose (Lösungsenthalpie)** $E_c$:

Die Bestimmung der Lösungsenthalpie erfolgt mittels eines Lösungskalorimeter 2225 *Precision Solution Calorimeter* der Fa. Thermometric.
Die Lösungswärme wird anhand der - infolge des Löseprozesses - auftretende Temperaturänderung und der aus der Basislinie berechneten systembedingten Temperaturänderung berechnet. Vor und nach dem Ampullenbruch wird jeweils eine elektrische Kalibrierung mit einem integrierten Heizwiderstand genau bekannter Leistung durchgeführt. Hierbei wird eine bekannte Wärmeleistung über einen festgelegten Zeitraum an das System abgegeben und der Temperatursprung ermittelt.

Messgerät und Einstellungen

| | |
|---|---|
| Lösungskalorimeter: | 2225 Precision Solution Calorimeter, Fa. Thermometric |
| Reaktionszelle: | 100 ml |
| Thermistorwiderstand: | 30,0 kΩ (bei 25 °C) |
| Rührergeschwindigkeit: | 500 U/min |
| Thermostat: | Thermostat des 2277 Thermal Activity Monitor TAM, Fa. Thermometric |
| Temperatur: | 25 °C ± 0.0001 °C (über 24h) |
| Meßampullen: | Crushing ampoules 1 ml, Fa. Thermometric |

(fortgesetzt)

| | |
|---|---|
| Dichtung: | Silikonstopfen und Bienenwachs, Fa. Thermometric |
| Einwaage: | 40 bis 50 mg |
| Lösemittel: | Wasser, chemisch rein |
| Volumen Lösemittel: | 100 ml |
| Badtemperatur: | 25°C |
| Temperaturauflösung: | High |
| Starttemperatur: | -40mK (± 10mK) temperature-offset |
| Interface: | 2280-002 TAM accessory interface 50 Hz, Fa. Thermometric |
| Software: | SolCal V 1.1 für WINDOWS |
| Auswertung: | Automatische Auswertung mit Menüpunkt CALCULATION/ ANALYSE EXPERIMENT. (Dynamik der Basislinie ; Kalibrierung nach dem Ampullenbruch). |

Elektrische Kalibrierung:

Die elektrische Kalibrierung erfolgt während der Messung, einmal vor und einmal nach dem Ampullenbruch. Zur Auswertung wird die Kalibrierung nach dem Ampullenbruch herangezogen.

| | |
|---|---|
| Wärmemenge: | 2,5 J |
| Heizleistung: | 500 mW |
| Heizdauer: | 10 s |
| Dauer der Basislinien: | 5 min (vor und nach Heizen) |

**V) Formulierungsbeispiele**

A) Inhaltionspulver:

1)

| Bestandteile | µg pro Kapsel |
|---|---|
| Tiotropiumbromidmonohydrat | 10,8 |
| **2a**'-Hydrochlorid | 35 |
| Lactose | 4954,2 |
| Summe | 5000 |

2)

| Bestandteile | µg pro Kapsel |
|---|---|
| Tiotropiumbromidmonohydrat | 21,7 |
| **2a'**-Maleatsalz | 75 |
| Lactose | 4903,3 |
| Summe | 5000 |

3)

| Bestandteile | µg pro Kapsel |
|---|---|
| Tiotropiumbromidmonohydrat | 22,5 |

(fortgesetzt)

| Bestandteile | µg pro Kapsel |
|---|---|
| **2a'**-Maleatsalz | 80,5 |
| Lactose | 4897 |
| Summe | 5000 |

4)

| Bestandteile | µg pro Kapsel |
|---|---|
| Tiotropiumbromid x H2O | 22,5 |
| **2a'**-Maleatsalz | 95,5 |
| Lactose | 4828 |
| Summe | 5000 |

B) Treibgashaltige Inhaltionsaerosole:

1)

| Bestandteile | Gew-% |
|---|---|
| Tiotropiumbromidmonohydrat | 0,015 |
| **2a**'-Hydrochlorid | 0,066 |
| Sojalecithin | 0,2 |
| TG134a: TG227 = 2:3 | ad 100 |

2)

| Bestandteile | Gew- % |
|---|---|
| Tiotropiumbromid | 0,029 |
| Verbindung **2a'**-Maleatsalz | 0,033 |
| Ethanol, absolut | 0,5 |
| Isopropylmyristat | 0,1 1 |
| TG 227 | ad 100 |

## Patentansprüche

1. Arzneimittelkombinationen **gekennzeichnet durch** den Gehalt an einem oder mehreren, bevorzugt einem Tiotropiumsalz **1** in Kombination mit einem pharmakologisch verträglichen Salz **2a** einer Verbindung der Formel **2a'**

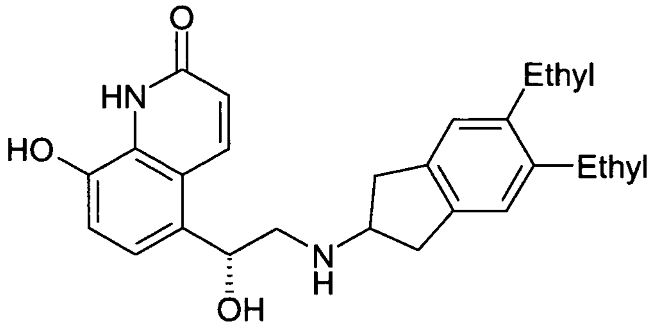

**2a'**

2. Arzneimittel nach Ansprch 1, **dadurch gekennzeichnet, daß** **1** in Form des Chlorids, Bromids, Iodids, Methansulfonats, para-Toluolsulfonats oder Methylsulfats, bevorzugt in Form des Bromids enthalten ist.

3. Arzneimittel nach einem der Ansprüche 1 oder 2, **dadurch gekennzeichnet, daß** die Wirkstoffe **1** und **2a** entweder gemeinsam in einer einzigen Darreichungsform oder in zwei getrennten Darreichungsformen enthalten sind.

4. Arzneimittel nach einem der Ansprüche 1 bis 3, **dadurch gekennzeichnet, daß** die Gewichtsverhältnisse von **1** zu **2a** in einem Bereich von 1:300 bis 30:1, bevorzugt von 1:230 bis 20:1 liegen.

5. Arzneimittel nach einem der Ansprüche 1 bis 4, **dadurch gekennzeichnet, daß** eine einmaliger Applikation einer Dosierung der Wirkstoffkombination **1** und **2a** von 0,01 bis 10000$\mu$g, bevorzugt von 0,1 bis 2000$\mu$g entspricht.

6. Arzneimittel nach einem der Ansprüche 1 bis 5, **dadurch gekennzeichnet, daß** es sich um eine Darreichungsform ausgewählt aus der Gruppe Inhalationspulver, treibgashaltige Dosieraerosole und treibgasfreie Inhalationslösungen oder -suspensionen handelt.

7. Arzneimittel nach Anspruch 6, **dadurch gekennzeichnet, daß** es ein Inhalationspulver ist, welches **1** und **2a** im Gemisch mit geeigneten physiologisch unbedenkliche Hilfsstoffen ausgewählt aus der Gruppe bestehend aus Monosaccharide, Disaccharide, Oligo- und Polysaccharide, Polyalkohole, Salze, oder Mischungen dieser Hilfsstoffe miteinander enthält.

8. Arzneimittel nach Anspruch 6, **dadurch gekennzeichnet, daß** es sich um ein treibgashaltiges Inhalationsaerosol handelt, welches **1** und **2a** in gelöster oder dispergierter Form enthält.

9. Arzneimittel nach Anspruch 8, **dadurch gekennzeichnet, daß** es sich um eine treibgasfreie Inhalationslösung oder -suspension handelt, die als Lösemittel Wasser, Ethanol oder ein Gemisch aus Wasser und Ethanol enthält.

10. Verwendung einer Zusammensetzung nach einem der Ansprüche 1 bis 9 zur Herstellung eines Medikaments zur Behandlung von entzündlichen oder obstruktiven Atemwegserkrankungen.

## Claims

1. Pharmaceutical combinations, **characterised in that** they contain one or more, preferably one tiotropium salt **1** combined with a pharmacologically acceptable salt **2a** of a compound of formula **2a'**

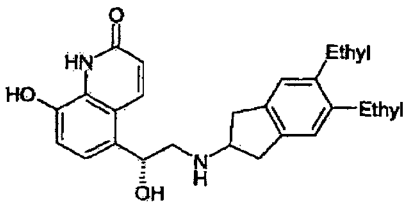

**2a'**

2. Pharmaceutical composition according to claim 1, **characterised in that** is present in the form of the chloride, bromide, iodide, methanesulphonate, paratoluene sulphonate or methyl sulphate, preferably in the form of the bromide.

3. Pharmaceutical composition according to one of claims 1 or 2, **characterised in that** the active substances 1 and 2a are either present together in a single preparation or are contained in two separate preparations.

4. Pharmaceutical composition according to one of claims 1 to 3, **characterised in that** the weight ratios of 1 to 2a are in the range from 1:300 to 30:1, preferably from 1:230 to 20:1.

5. Pharmaceutical composition according to one of claims 1 to 4, **characterised in that** a single application corresponds to a dosage of the combination of active substances 1 and 2a of 0.01 to 10000 μg, preferably from 0.1 to 2000 μg.

6. Pharmaceutical composition according to one of claims 1 to 5, **characterised in that** it is a formulation selected from among inhalable powders, propellant-containing metered-dose aerosols and propellant-free inhalable solutions or suspensions.

7. Pharmaceutical composition according to claim 6, **characterised in that** it is an inhalable powder which contains 1 and 2a in admixture with suitable physiologically acceptable excipients selected from among the monosaccharides, disaccharides, oligo- and polysaccharides, polyalcohols, salts, or mixtures of these excipients with one another.

8. Pharmaceutical composition according to claim 6, **characterised in that** it is a propellant-containing inhalable aerosol which contains 1 and 2a in dissolved or dispersed form.

9. Pharmaceutical composition according to claim 8, **characterised in that** it is a propellant-free inhalable solution or suspension which contains water, ethanol or a mixture of water and ethanol as solvent.

10. Use of a composition according to one of claims 1 to 9 for preparing a medicament for treating inflammatory or obstructive diseases of the respiratory tract.

## Revendications

1. Combinaisons médicamenteuses **caractérisées par** la teneur en un ou plusieurs, de préférence un sel de tiotropium 1 en combinaison avec un sel pharmacologiquement acceptable 2a d'un composé de formule 2a'

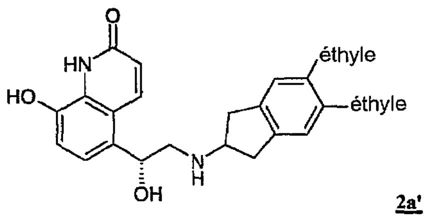

2a'

2. Médicament selon la revendication 1 **caractérisé en ce que** 1 est contenu sous forme de chlorure, de bromure, d'iodure, de méthanesulfonate, de para-toluènesulfonate ou de méthylsulfate, de préférence sous forme de bromure.

3. Médicament selon l'une des revendications 1 ou 2 **caractérisé en ce que** les principes actifs 1 et 2a sont contenus soit ensemble dans une forme d'administration unique soit dans deux formes d'administration séparées.

4. Médicament selon l'une des revendications 1 à 3 **caractérisé en ce que** les proportions massiques de 1 à 2a sont situées dans un domaine de 1 : 300 à 30 : 1, de préférence de 1 : 230 à 20 : 1.

5. Médicament selon l'une des revendications 1 à 4 **caractérisé en ce qu'**une application unique correspond à une posologie de la combinaison de principes actifs 1 et 2a de 0,01 à 10000 μg, de préférence de 0,1 à 2000 μg.

6. Médicament selon l'une des revendications 1 à 5 **caractérisé en ce qu'**il s'agit d'une forme d'administration choisie

dans le groupe poudres pour l'inhalation, aérosols de dosage contenant un gaz propulseur et solutions ou suspensions pour l'inhalation sans gaz propulseur.

7. Médicament selon la revendication 6 **caractérisé en ce que** c'est une poudre pour l'inhalation qui contient **1** et **2a** en mélange avec des adjuvants physiologiquement acceptables appropriés choisis dans le groupe consistant en les monosaccharides, les disaccharides, les oligo- et polysaccharides, les polyalcools, les sels, ou les mélanges de ces adjuvants entre eux.

8. Médicament selon la revendication 6 **caractérisé en ce qu'**il s'agit d'un aérosol pour l'inhalation contenant un gaz propulseur qui contient **1** et **2a** sous forme dissoute ou dispersée.

9. Médicament selon la revendication 8 **caractérisé en ce qu'**il s'agit d'une solution ou suspension pour l'inhalation sans gaz propulseur qui contient comme solvant de l'eau, de l'éthanol ou un mélange d'eau et d'éthanol.

10. Utilisation d'une composition selon l'une des revendications 1 à 9 pour la production d'un médicament pour le traitement des maladies inflammatoires ou obstructives des voies respiratoires.

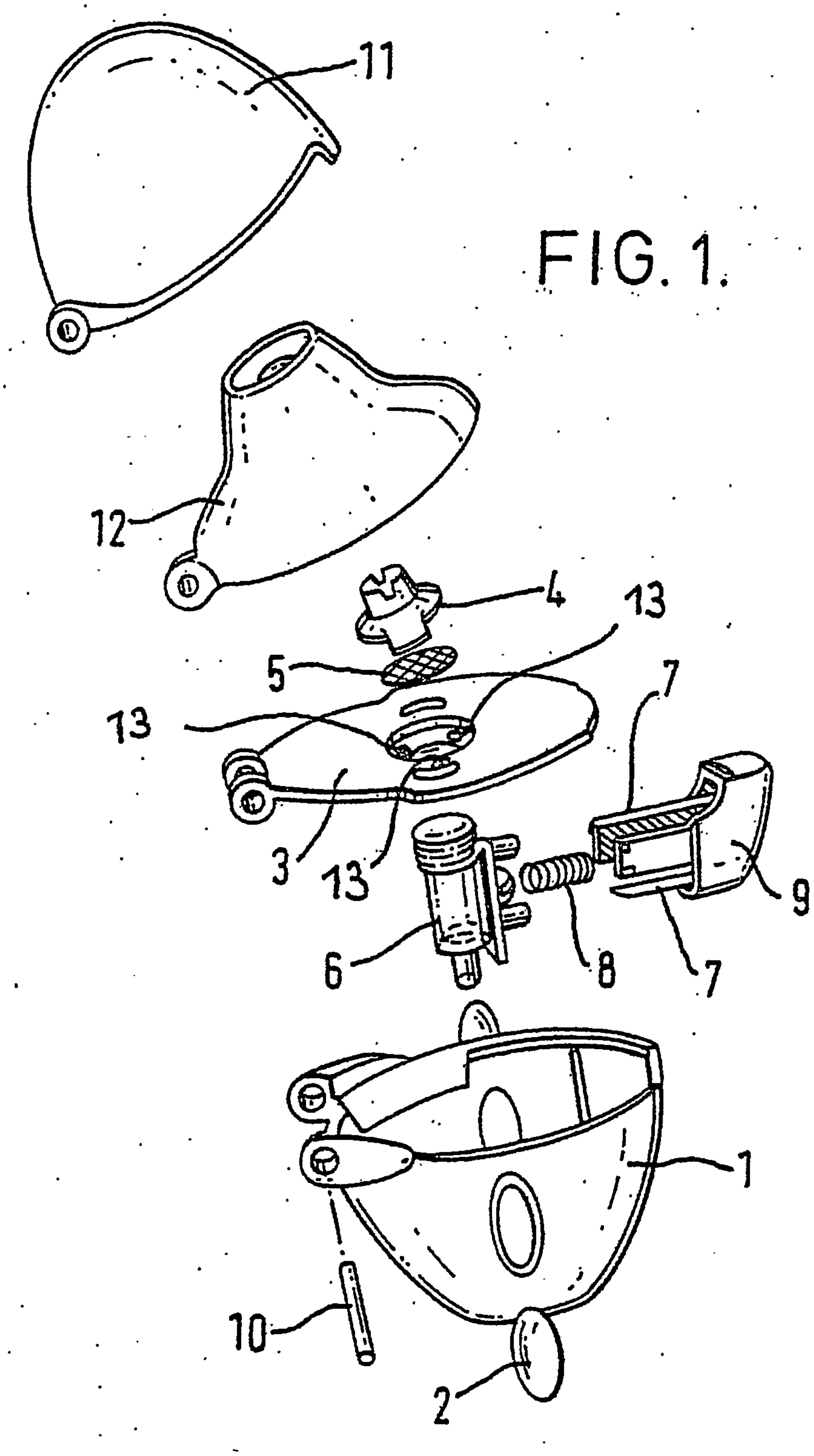


FIG. 1.

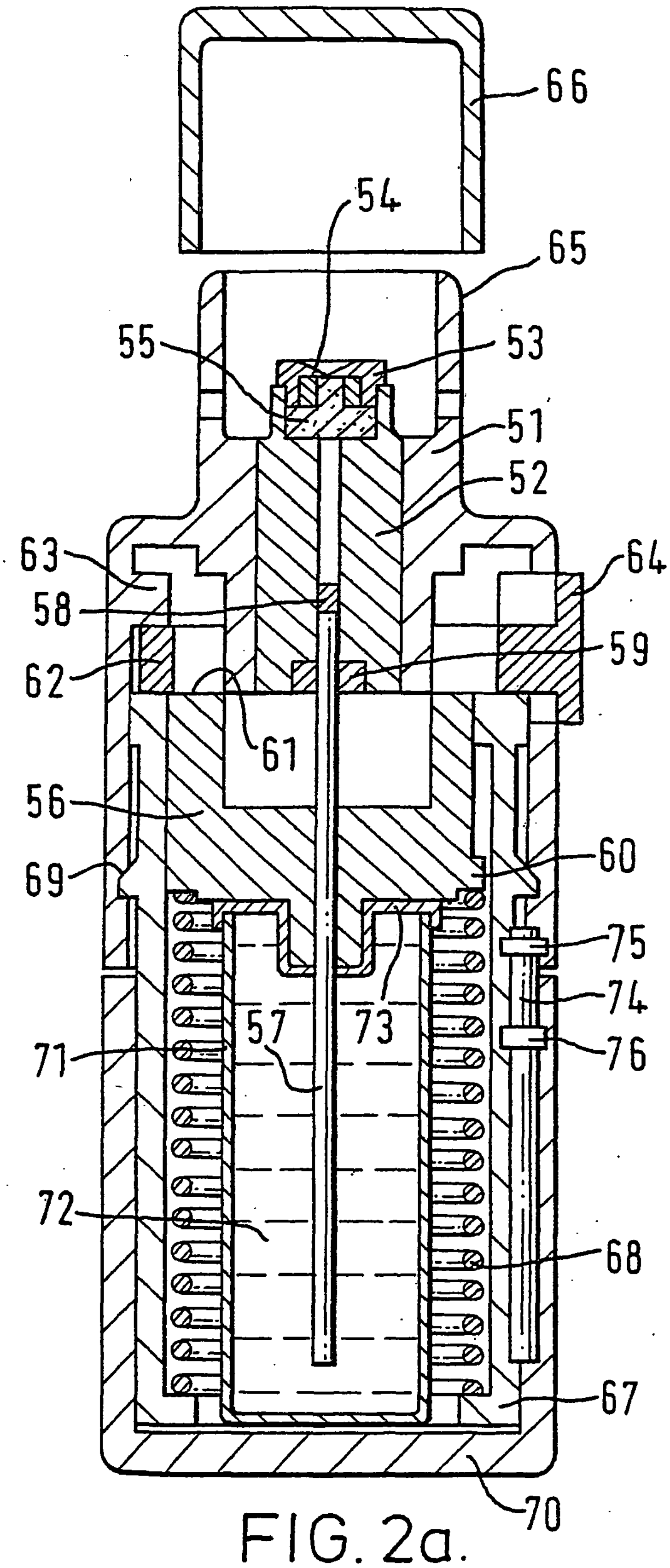
66
54
65
55
53
51
52
63
64
58
59
62
61
56
60
69
75
74
73
57
71
76
72
68
67
70

FIG. 2a.

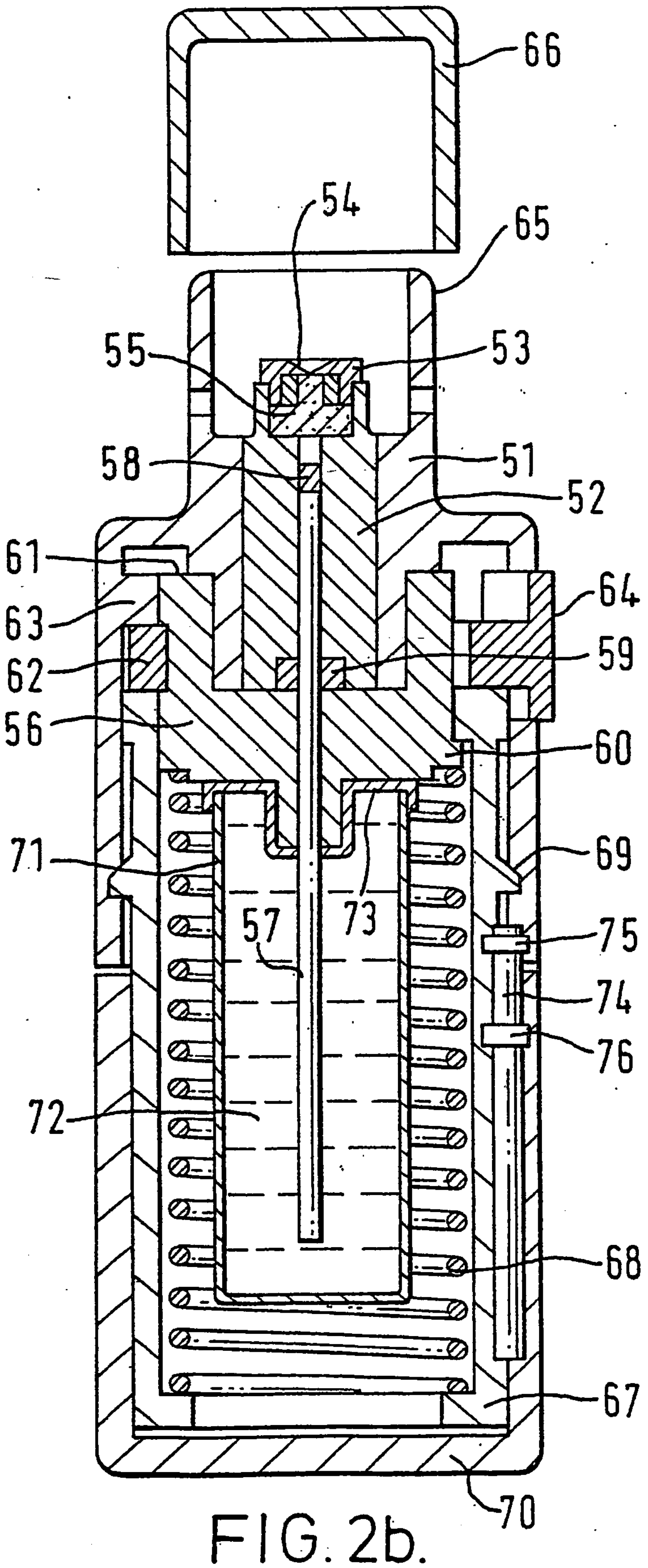
66
54
65
55
53
58
51
52
61
64
63
59
62
56
60
71
69
57
73
75
74
76
72
68
67
70

FIG. 2b.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 418716 A1
- WO 0075114 A
- WO 0230928 A
- WO 0230390 A
- US 4570630 A
- DE 3625685 A
- WO 9428958 A
- WO 9114468 A
- WO 9712687 A
- WO 9407607 A
- WO 9720590 A
- WO 9712683 A